Europäisches Patentamt

European Patent Office

Office européen des brevets

(19)

(11) Veröffentlichungsnummer: **0 238 914**
**B1**

(12) **EUROPÄISCHE PATENTSCHRIFT**

(45) Veröffentlichungstag der Patentschrift: **26.09.90**

(21) Anmeldenummer: **87103214.0**

(22) Anmeldetag: **06.03.87**

(51) Int. Cl.⁵: **C 12 Q 1/26,** G 01 N 33/72,
C 12 Q 1/37

(54) **Verfahren und Reagenz zur Bestimmung von Gesamt-Bilirubin.**

(30) Priorität: **14.03.86 DE 3608453**

(43) Veröffentlichungstag der Anmeldung:
**30.09.87 Patentblatt 87/40**

(45) Bekanntmachung des Hinweises auf die
Patenterteilung:
**26.09.90 Patentblatt 90/39**

(84) Benannte Vertragsstaaten:
**AT BE CH DE ES FR GB GR IT LI LU NL SE**

(56) Entgegenhaltungen:
EP-A-0 005 637
WO-A-86/00933
FR-A-2 521 589
GB-A-2 146 997

BIOLOGICAL ABSTRACTS, Band 74, Nr. 7, 1982,
Zusammenfassung Nr. 49336; P.M. HOROWITZ
et al.: "Controlled proteolysis by subtilisin as a
probe for cyanide-induced conformational
changes in Pseudomonas aeruginosa
cytochrome oxidase (EC 1.9.3.2)"

(73) Patentinhaber: **BOEHRINGER MANNHEIM
GMBH
Sandhofer Strasse 116
D-6800 Mannheim 31 (DE)**

(72) Erfinder: **Kruse-Müller, Cornelia, Dr. rer. nat.
Kellerwiese 10
D-8132 Tutzing (DE)**
Erfinder: **Siedel, Joachim, Dr. rer. nat.
Bahnhofstrasse 6
D-8131 Bernried (DE)**
Erfinder: **Ziegenhorn, Joachim, Dr. rer. nat.
Ina-Seidel-Weg 1
D-8130 Starnberg (DE)**

(56) Entgegenhaltungen:

BIOLOGICAL ABSTRACTS, Band 76, Nr. 9, 1983,
Zusammenfassung Nr. 67710; P.M. HOROWITZ
et al.: "Activation of Pseudomonas aeruginosa
cytochrome oxidase (EC 1.9.3.2) by Munited
procteolysis with subtilisin"

**Beschreibung**

Die Erfindung betrifft ein Verfahren sowie ein Reagenz zur Bestimmung von Gesamt-Bilirubin mittels Bilirubin-Oxidase.

Im klinisch-chemischen Labor ist die Bilirubin-Bestimmung, insbesondere im Serum, ein wichtiger Parameter zur Abklärung diverser Krankheitsbilder, z. B. bei Hepatitis, Leberzirrhose, Leber-/Gallen- und hämolytischen Erkrankungen. Beim Neugeborenen tritt in den ersten Lebenstagen ein sogenannter physiologischer Ikterus auf, der bei zu hohen Bilirubinwerter letale oder zumindest hirnschädigende Auswirkungen hat (Kernikterus).

Bilirubin kann im Serum an Albumin adsorptiv (α-Bilirubin), an einen Zuckerrest (β-Bilirubin), an zwei Zuckerreste (γ-Bilirubin) oder kovalent an Albumin gebunden (δ-Bilirubin) vorliegen. β-plus γ-Bilirubin werden als konjugiertes Bilirubin, α-Bilirubin wird als unkonjugiertes Bilirubin bezeichnet. Serum-Gesamt-Bilirubin setzt sich aus den Fraktionen α- bis δ-Bilirubin zusammen. Eine umfassende Darstellung hierzu findet sich in Clinical Biochermistry 17 (1984), 221 - 229.

Die Bestimmung von Gesamt-Bilirubin kann nach dem von Jendrassik beschriebenen Verfahren (Jendrassik, Biochem. Z. 297 (1938), 81) durchgeführt werden. Hierbei wird Bilirubin in Gegenwart von Coffein mit diazotierter Sulfanilsäure zu einem Azofarbstoff gekuppelt. Dieses Verfahren hat etliche Nachteile: Einmal ist das Gebrauchsreagenz sehr instabil und muß unmittelbar vor der Bestimmung hergestellt werden. Damit ist der Test in der Handhabung umständlich und nur schwer automatisierbar. Weiterhin führt die Anwesenheit von Nitrit im Jendrassik-Test sowie der stark saure pH in diesem wie auch anderen Tests mit Diazoniumsalzen zu Störungen bei hämolytischen Seren.

Eine weitere Methode zur Bestimmung von Gesamt-Bilirubin ist in Scand. J. Clin. Lab. Invest. 29, Suppl. 126 (1972), Abstract 11.12, beschrieben. Hierbei wird Bilirubin mit einer Diazoniumverbindung zu einem Azo-Bilirubin gekoppelt und die Farbbildung gemessen. Die Freisetzung des unkonjugierten Bilirubins erfolgt durch Detergenz-Zusatz. Ebenso wie die Jendrassik-Methode ist diese Bestimmung in hämolytischen Seren gestört. Da auch andere Bestandteile des Serums, wie z. B. Indican, mit Diazoniumsalzen reagieren können, werden in Urämikerseren oft zu hohe Werte für Gesamt-Bilirubin gefunden.

Ein weiteres Verfahren zur Bestimmung von Gesamt-Bilirubin ist in Selected Topics in Clinical Enzymology 2 (1984), 97 - 107 beschrieben. Danach wird Bilirubin mit Bilirubin-Oxidase (E.C. 1.3.3.5) umgesetzt und die Extinktionsabnahme von Bilirubin bei 410 - 480 nm gemessen. Dieses Verfahren wird durch hämolytische bzw. Urämikerseren nicht gestört. Es hat jedoch den Nachteil, daß der Endpunkt der Reaktion, in Abhängigkeit von der jeweils eingesetzten Probe, oft erst nach 1 - 2 Std. erreicht wird. Der wesentliche Grund dafür dürfte darin liegen, daß das Albumin-gebundene Bilirubin einer enzymatischen Umsetzung kaum zugänglich ist und seine Dissoziation vom Albumin nur sehr langsam oder (im Fall von δ-Bilirubin) überhaupt nicht abläuft. Damit ist auch dieses Verfahren für die Routineanalyse im Labor, insbesondere für die Verwendung an automatischen Systemen, ungeeignet.

Es wurde deshalb mehrfach versucht, dieses Verfahren so zu verbessern, daß auch Albumingebundenes Bilirubin vollständig miterfaßt wird.

Aus DE 32 39 236 ist ein Verfahren zur Bestimmung von Bilirubin mit Bilirubin-Oxidase, unter Zusatz von oberflächenaktiven Mitteln, aromatischen Carbonsäuren, Sulfanilamiden oder Proteasen (Pronase P) bekannt. Die Verwendung von Proteasen -und insbesondere von Protease-Gemischen wie Pronase P- haben hierbei den erheblichen Nachteil, daß diese Enzyme das Protein Bilirubin-Oxidase abbauen und damit je nach Dauer der Lagerung des Reagenzes oder der Inkubationszeit eine Verringerung der Bilirubin-Oxidase-Aktivität bewirken. Eine Überprüfung dieses Verfahrens ergab, daß es damit nicht gelingt, Bilirubin vollständig zu erfassen.

Aus Chemical Abstracts, CA 102: 20074e ist bekannt, die Probe mit oberflächenaktiven Mitteln, sowie einer nicht näher spezifizierten protease und/oder Lipase vorzubehandeln, um die Bindung zwischen Bilirubin und Albumin zu lösen. Hiernach gelingt es, mit Natrium-Laurylsulfat (Natrium-Dodecylsulfat, SDS) zwar wesentlich mehr Bilirubin (89 % des in der Probe enthaltenen Gesamtbilirubins) als ohne solche Zusätze (19 %) zu erfassen, aber eben auch nicht komplett. Somit ist dieses Verfahren für einen quantitativen Test, der auch für Proben mit unterschiedlichem Gehalt an Albumin-gebundenem Bilirubin anwendbar ist, ebenfalls nicht geeignet.

In Selected Topics in Clinical Enzymology 2, (1984), 97 - 107, Walter de Gruyter & Co., Berlin/New York, wird vorgeschlagen, die Bestimmung in Gegenwart von anionischen Detergentien, wie Natrium-Dodecylsulfat, durchzuführen. Zur vollständigen Freisetzung von albumingebundenem Bilirubin sind jedoch sehr hohe Konzentrationen an Detergenz (300 Mol SDS/Mol Albumin) erforderlich, was andererseits jedoch schon nach kürzester Zeit die Bilirubin-Oxidase weitgehend inaktiviert. Mit geringeren Detergenzkonzentrationen wird zwar eine Verbesserung der Albuminablösung im Vergleich zu einem detergenzfreien Reagenz gefunden, jedoch wird die vollständige Freisetzung des Albumin-gebundenen Bilirubins nicht innerhalb einer für die Routineanalytik zumutbaren Inkubationszeit (z. B. unter 30 min) erreicht.

Bei Verwendung derartiger Detergentien zeigt sich, daß außerdem probenabhängig ein erheblicher bis zu 2 Std. dauernder Reaktionsschleich auftritt. Dies verhindert ebenfalls die Verwendung des Verfahrens zur enzymatischen Bestimmung von Gesamtbilirubin in der Routinediagnostik.

Aufgabe der vorliegenden Erfindung war daher, ein Verfahren zur Bestimmung von Gesamt-Bilirubin zu entwickeln, welches die o. g. Nachteile nicht aufweist, einfach durchführbar und automatisierbar ist und auch bei hohem Anteil von Albumin-gebundenem Bilirubin in der Probe richtige Ergebnisse liefert.

Diese Aufgabe wird erfindungsgemäß gelöst durch ein Verfahren zur Bestimmung von Gesamt-Bilirubin in Körperflüssigkeiten, mittels Bilirubin-Oxidase, dadurch gekennzeichnet, daß die Probe mit Subtilisin inkubiert wird.

Subtilisin (EC 3.4.21.14) umfaßt Serin-Proteasen, welche beispielsweise aus Bacillus subtilis (Subtilopeptidase A) oder verwandten Bakterien (Subtilopeptidase B und C) isoliert werden können (M. Ottesen, I. Svendsen 1970, Methods in Enzymology 19, 199 - 215).

Daß mit dem erfindungsgemäßen Verfahren Gesamt-Bilirubin bestimmt werden kann, ist besonders überraschend, da es durch Zusatz von anderen Proteasen, wie z. B. Pronase P, Endoproteinase Arg C, Papain, Bromelain, Trypsin oder Chymotrypsin, nicht gelingt, Bilirubin vollständig zu erfassen. Außerdem wird bei Verwendung dieser Proteasen ein Reaktionsschleich sowie eine Desaktivierung der Bilirubin-Oxidase, vermutlich durch Proteolyse, beobachtet.

Dagegen setzt Subtilisin in überraschend kurzer Zeit Bilirubin aus seiner Albuminbindung vollständig frei, und es tritt nach kurzer Zeit (ca. 1 min) auch keinerlei Reaktionsschleich mehr auf.

Außerdem wird Bilirubin-Oxidase trotz der Protease-Aktivität von Subtilisin erstaunlicherweise nicht desaktiviert, zumindest nicht in einem Zeitraum von 30 min bis 37°C. Die Gründe hierfür sind nicht bekannt.

Die Durchführung des erfindungsgemäßen Verfahrens erfolgt in der Weise, daß die Probe (beispielsweise Plasma oder Serum) mit Subtilisin inkubiert wird. Die Inkubationsdauer kann hierbei in weiten Bereichen variiert werden. Die erforderliche minimale Inkubationszeit ist im wesentlichen von der Temperatur abhängig und beträgt beispielsweise bei 20°C nicht mehr als ca. 5 min und bei 37°C ca. 3 min. Längere Inkubationszeiten, wie z. B. 20 oder 30 min, sind jedoch ebenfalls möglich, ohne daß sich die Meßergebnisse verändern.

Nach erfolgter Inkubation wird Bilirubin-Oxidase zugegeben und Gesamt-Bilirubin bestimmt. Zur Bestimmung kommen insbesondere in Frage:

a) Messung der Extinktionsabnahme der Bilirubin-Absorptionsbande (zwischen ca. 410 bis 480 nm), (Selected Topics, s. o., oder EP 0 005637)

b) Messung des Sauerstoffverbrauchs während der Bilirubin-Oxidation,

c) Messung der Farbstoffbildung in Gegenwart von MBTH (s. DEA 34 36 005, Takara-Shuzo).

Wird statt einer $H_2O$- eine $H_2O_2$-liefernde Bilirubin-Oxidase eingesetzt, kann der Bestimmung auch die Messung von $H_2O_2$ zugrundegelegt werden, z. B. colorimetrisch durch die Katalasekatalysierte Bildung von Formaldehyd aus Methanol und Erzeugung eines Dihydrolutidinfarbstoffes aus besagtem Formaldehyd in Gegenwart von Ammoniumionen und Acetylaceton, oder durch die oxidative Kupplung von MBTH-S mit Phenolen, Anilinen, Naphtholen der Naphthylamine in Gegenwart von Peroxidase. Besonders bevorzugt erfolgt die Bestimmung nach a).

In einer bevorzugten Ausführungsform werden Subtilisin und Bilirubin-Oxidase gleichzeitig mit der Probe inkubiert. Der Bilirubingehalt wird dann beispielsweise durch die Differenz zwischen Anfangsextinktion und der nach der Inkubation gemessenen Extinktion bestimmt; zur Eichung wird ein Standard mit bekanntem Bilirubingehalt mitgeführt.

Art und Konzentration der Reaktionspartner sowie Reaktionsbedingungen, wie pH-Wert und Pufferkapazitäten beim erfindungsgemäßen Verfahren, entsprechen den nachfolgend beschriebenen Angaben zum erfindungsgemäßen Reagenz.

Ein weiterer Gegenstand der Erfindung ist ein Reagenz zur Bestimmung von Gesamt-Bilirubin, welches dadurch gekennzeichnet ist, daß es aus einer wäßrigen, gepufferten Lösung besteht, welche Bilirubin-Oxidase und, gegebenenfalls getrennt davon, Subtilisin enthält.

Der pH-Wert der wäßrigen Lösungen liegt zweckmäßig zwischen 4,5 und 9,5, vorzugsweise zwischen 6 und 9. Zur Einstellung des pH-Werts kommen alle Puffersubstanzen in Frage, deren pK-Werte so liegen, daß sie in dem genannten pH-Bereich ausreichende pufferkapazität aufweisen. Besonders geeignet sind Phosphat-, Tris-, Tricin-, Hepes-, MES- und Acetat-Puffer. Die Konzentration an Puffersubstanz beträgt zweckmäßig 0,01—1 Mol/1, bevorzugt sind 0,05 0,2 Mol/1. Diese Konzentrationsangaben sowie alle anderen hier erwähnten Konzentrationsangaben, incl. der Enzymaktivitäten, sind Endkonzentrationen im Test.

Subtilisin wird zu einer Aktivität von 1 - 50 U/ml, bevorzugt 2 - 20 U/ml, besonders bevorzugt 5 - 10 U/ml eingesetzt.

Bilirubin-Oxidase (beziehbar z. B. von Takara Shuzo, Japan) wird zu einer Aktivität von 0,005 - 20 U/ml, bevorzugt 0,05 - 5 U/ml eingesetzt.

Ein bevorzugtes Reagenz gem. der Erfindung enthält in einer gepufferten, wäßrigen Lösung

0,05 5 U/ml Bilirubin-Oxidase

0,05 0,2 Mol/1 Puffersubstanz, pH 6 - 9

und ggf. getrennt davon

2 - 20 U/ml Subtilisin.

In einer weiteren Ausführungsform liegen die genannten Reaktionskomponenten auf/oder in mindestens einem Trägermaterial imprägniert vor. Als Trägermaterial kann ein saugfähiges oder quellfähiges oder filmbildendes Trägermaterial, z. B. ein für Teststreifen bekanntes Trägermaterial, wie Papier und ähnliche Vliesmaterialen, wie beispielsweise Teebeutelpapier oder Glasfaservlies, verwendet werden. Dabei können die Reaktionskomponenten auf mehrere miteinander in Kontakt stehende Träger verteilt werden. Als besonders vorteilhaft hat es sich erwiesen, wenn der erste Träger Subtilisin und ein weiterer, damit in Kontakt stehender Träger Bilirubin-Oxidase enthält.

Die folgenden Beispiele und Abbildungen erläutern die Erfindung weiter.

Figur 1 zeigt einen Methodenvergleich zwischen dem erfindungsgemäßen Verfahren und der DPD-Methode (Scand. J. Clin. Lab. Invest., siehe oben).

Figur 2 zeigt einen Vergleich des Reaktionsschleichs für die Bilirubinbestimmung mittels Bilirubin-Oxidase bei Zusatz von Proteasen/Detergens.

Beispiel 1

Bestimmung von Gesamt-Bilirubin (1-Schritt-Test) 30 µl Probe (Serum oder Standard) und 500 µl Puffer (0,05 mol/1 Na-phosphat pH 7,5) werden zusammenpipettiert. Die Extinktion der Lösung wird bei 436 nm bestimmt. Die Reaktion wird durch Zugabe von 50 µl eines Gemisches aus Subtilisin und Bilirubin-Oxidase gestartet (SubtilisinEndkonzentration: 10 U/ml, Bilirubin-Oxidase-Endkonzentration: 0,1 U/ml).

Nach 10minütiger Inkubation bei 37°C wird erneut die Extinktion bei 436 nm bestimmt. Aus der Differenz von Anfangs und Endextinktion wird die Bilirubinkonzentration der Probe über einen Faktor, der mittels eines mitgeführten Standards bekannten Bilirubingehalts bestimmt wird, errechnet.

Beispiel 2

Bestimmung von Gesamt-Bilirubin (2-Schritt-Test) Zu 30 µl Probe (Serum oder Standard) und 500 µl Puffer (0,05 mol/1 NaPhosphat, pH 7,5) werden 20 µl Subtilisinlösung (Endkonzentration 10 U/ml) pipettiert. Nach 10minütiger Inkubation bei 37°C wird die Extinktion bei 436 nm bestimmt. Die Reaktion wird durch Zugabe von 20 µl Bilirubin-Oxidase (Endkonzentration 0,1 U/ml) gestartet. Nach 5 min wird erneut die Extinktion bei 436 nm abgelesen und aus der Differenz zwischen Anfangs- und Endextinktion der Bilirubingehalt der Probe über einen Faktor, der mittels eines mitgeführten Standards bekannten Bilirubingehalts bestimmt wird, errechnet.

Beispiel 3

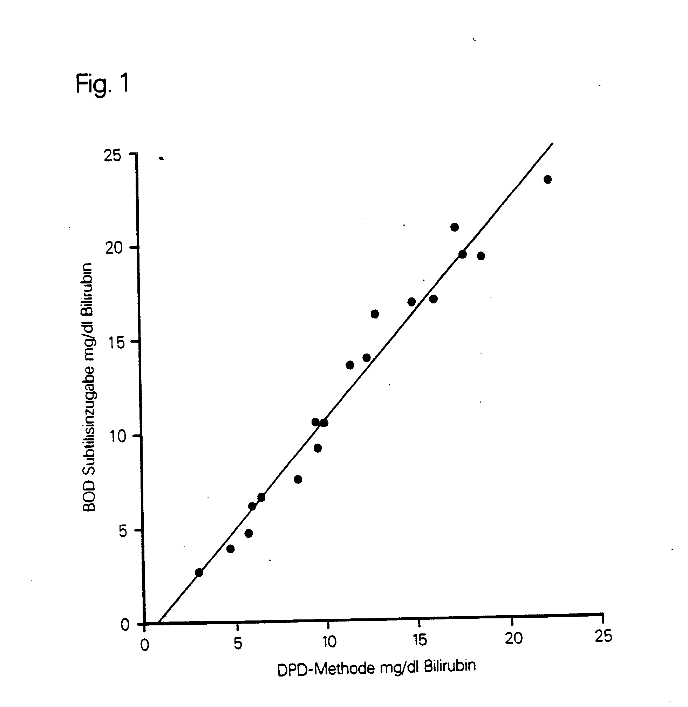

Vergleich mit DPD-Methode Mit verschiedenen Proben (Serum) wurde Bilirubin gemäß Beispiel 2 und nach der DPD-Methode (Monotest® 10 BOEHRINGER MANNHEIM GmbH, Best.-Nr. 12394) verglichen. Das Ergebnis zeigt Fig. 1. Der Korrelationskoeffizient beträgt R = 0,985. Als Regressionsgleichung ergab sich y = 1,13 x − 0824.

Beispiel 4

Meßwertänderung bei Verwendung verschiedener Proteasen

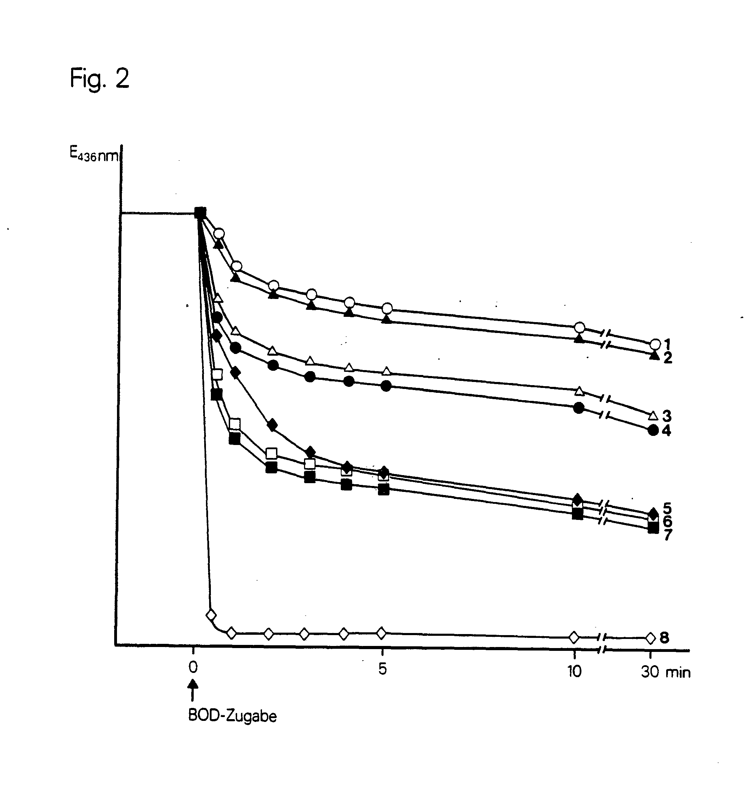

Bei einer Bestimmung gemäß Beispiel 2 wurden an Stelle von Subtilisin verwendet (Fig. 2):

Kurve 1: ohne Proteasen
Kurve 2: Endo-Protease Arg C 4mg/ml
Kurve 3: Papain 4 mg/ml
Kurve 4: Bromelain 4 mg/ml
Kurve 5: Trypsin 20 mg/ml
Kurve 6: Chymotrypsin 4 mg/ml
Kurve 7: SDS 0,1 %
Kurve 8: Beispiel 2 (Vergleichsmessung)

Aus Fig. 2 ergibt sich, daß mit SDS die erfindungsgemäße Wirkung nicht erreicht werden kann. Mit anderen Serinproteasen, wie z. B. Trypsin oder Chymotrypsin, kann keine merkliche Verringerung des Reaktionsschleichs erreicht werden. Außerdem haben diese Proteasen den Nachteil, daß sie zusätzlich noch die Bilirubin-Oxidase angreifen und damit das Meßergebnis verfälschen.

**Patentansprüche**

1. Verfahren zur Bestimmung von Gesamt-Bilirubin in Proben von Körperflüssigkeiten mittels Bilirubin-Oxidase, dadurch gekennzeichnet, daß die Probe mit Subtilisin inkubiert wird.

2. Verfahren nach Anspruch 1, dadurch gekennzeichnet, daß die Inkubation mit Subtilisin in Gegenwart von Bilirubin-Oxidase erfolgt.

3. Verfahren nach Anspruch 1 und 2, dadurch gekennzeichnet, daß man Subtilisin in einer Aktivität von 1 50 U/ml einsetzt.

4. Verfahren nach Anspruch 1 bis 3, dadurch gekennzeichnet, daß die Inkubation in wäßriger, gepufferter Lösung erfolgt.

5. Reagenz zur Bestimmung von Gesamt-Bilirubin in Proben von Körperflüssigkeiten, in Gegenwart von Bilirubin-Oxidase, dadurch gekennzeichnet, daß es

0,005 - 20 U/ml Bilirubin-Oxidase
0,01 - 1,0 Mol/l Puffersubstanz, pH 4 - 9,5 und ggf. davon getrennt
1 - 50 U/ml Subtilisin enthält.

6. Reagenz nach Anspruch 5, dadurch gekennzeichnet, daß es

0,05 - 5 U/ml Bilirubin-Oxidase
0,05 - 0,2 Mol/l Puffersubstanz, pH 4 - 9,5 und, ggf. davon getrennt
2 - 20 U/ml Subtilisin enthält.

7. Reagenz nach Anspruch 5 oder 6, dadurch gekennzeichnet, daß es die Reaktionskomponenten auf oder in mindestens einem saugfähigen, quellfähigen oder filmbildenden Trägermaterial enthält.

**Revendications**

1. Procédé pour la détermination de la bilirubine totale dans des échantillons de liquides corporels au moyen de la bilirubine oxydase, caractérisé en ce que l'on fait incuber les échantillons avec la subtilisine.

2. Procédé selon la revendication 1, caractérisé en ce que l'incubation avec la subtilisine s'effectue en présence de bilirubine oxydase.

3. Procédé selon les revendications 1 et 2, caractérisé en ce que l'on utilise une subtilisine ayant une activité de 1—50 U/ml.

4. Procédé selon les revendications 1 à 3, caractérisé en ce que l'incubation est effectuée dans une solution aqueuse, tamponnée.

5. Réactif pour la détermination de la bilirubine totale dans des échantillons de liquides corporels, en présence de bilirubine oxydase, caractérisé en ce qu'il contient

0,005—20 U/ml de bilirubine oxydase

0,01—1,0 mole/litre de substance tampon, pH 4—9,5

et éventuellement séparément

1—50 U/ml de subtilisine.

6. Réactif selon la revendication 5, caractérisé en ce qu'il contient

0,05—5 U/ml de bilirubine-oxidase

0,05—0,2 mole/litre de substance tampon, pH 4—9,5

et éventuellement séparément

2—20 U/ml de subtilisine.

7. Réactif selon la revendication 5 ou 6, caractérisé en ce qu'il contient les composants réactionnels sur, ou dans, au moins une substance de support absorbante, susceptible de gonfler ou filmogène.

**Claims**

1. Process for the determination of total bilirubin in samples of body fluids by means of bilirubin oxidase, characterised in that the sample is incubated with subtilisin.

2. Process according to claim 1, characterised in that the incubation takes place with subtilisin in the presence of bilirubin oxidase.

3. Process according to claim 1 and 2, characterised in that one uses subtilisin with an activity of 1 - 50 U/ml.

4. Process according to claims 1 to 3, characterised in that the incubation takes place in aqueous, buffered solution.

5. Reagent for the determination of total bilirubin in samples of body fluids in the presence of bilirubin oxidase, which is characterised in that it contains

0.005 - 20 U/ml. bilirubin oxidase

0.01 - 1.0 mole/l. buffer substance, pH 4 - 9.5

and possibly separate therefrom

1 - 50 U/ml. subtilisin.

6. Reagent according to claim 5, characterised in that it contains

0.05 - 5 U/ml. bilirubin oxidase

0.05 - 0.2 mole/1. buffer substance, pH 4 - 9.5

and possibly separate therefrom

2 - 20 U/ml. subtilisin.

7. Reagent according to claim 5 or 6, characterised in that it contains the reaction components on or in at least one absorbent, swellable or film-forming carrier material.

Fig. 1

Fig. 2